# EUROPEAN PATENT APPLICATION

(11) **EP 4 717 766 A1**
(43) Date of publication of application: **01.04.2026**
(21) Application number: 24830792.8
(22) Date of filing: 26.06.2024
(51) Int. Cl.: C12N 7/04, C12N 7/01

(54) **DENDRITIC CELL-TARGETED VIROID DVLP, AND PREPARATION METHOD THEREFOR AND USE THEREOF**

(30) Priority: 27.06.2023 CN 202310772281; 20.06.2024 CN 202410801991
(71) Applicant: Shanghai Bdgene Co., Ltd, Shanghai 201111 (CN)
(72) Inventor: CAI, Yujia, Shanghai 200240 (CN)
(74) Representative: Bayramoglu et al.
(86) International application number: PCT/CN2024/101512
(87) International publication number: WO 2025/002153

(57) **Abstract**

A dendritic cell-targeted virus-like particle DVLP, and preparation method and application thereof. The virus-like particle DVLP is composed of a virus-like particle (VLP) encapsulated by an optimized Sindbis virus glycoprotein whose sequence is shown in SEQ ID NO.1. The preparation includes the following steps: optimizing the amino acid sequence of the envelope protein of wild-type Sindbis virus to obtain the SV-G gene sequence; constructing an expression vector of the SV-G gene, namely pCMV-SV-G; co-transfecting HEK293T cells with plasmids including pCMV-SV-G, pRSV-REV, an antigen expression vector, pMDlg/PRRE-D64V, and pMS2S-PH-gagpol-D64V, followed by transfection and purification. The virus-like particle DVLP has extremely high DC targeting ability and protein stability. After injection, the mRNA carried by the virus-like particle DVLP is mainly concentrated at the injection site and in lymph nodes, with a low risk of spreading to non-target organs such as the spleen, liver, and kidneys. Moreover, it can generate high-titer neutralizing antibodies to resist viral infection and protect the host, thereby providing a platform for the development of tumor vaccines.

## Description

### TECHNICAL FIELD

The present invention belongs to the field of biomedicine and relates to a dendritic cell-targeted virus-like particle DVLP, and preparation method and application thereof, and specifically relates to application of dendritic cell-targeted virus-like particle mRNA delivery technology in vaccines.

### BACKGROUND TECHNOLOGY

Dendritic cells (DCs) are important antigen-presenting cells (APCs) that play a crucial role in the efficacy of vaccines. Their functions are mainly reflected in the following two aspects: First, DCs can process and present antigens to T cells, thereby inducing cellular immunity; second, DCs can process and present antigens to B cells and induce humoral immune responses. In 2010, the U.S. Food and Drug Administration (FDA) approved PROVENGE, the world's first DC vaccine, for the treatment of prostate cancer. However, the production process of PROVENGE is complex and cumbersome, involving the isolation of the patient's autologous cells, *in vitro* preparation, and subsequent *in vivo* transfusion. Therefore, this vaccine has high production costs and selling prices, resulting in poor accessibility. In addition, the translation of antigen mRNA in non-professional APCs may cause cells expressing the antigen to become targets for CD8+ T cell-mediated killing, which is associated with the side effects observed in some people after receiving COVID-19 mRNA vaccines. Furthermore, antibody-dependent cellular cytotoxicity (ADCC) may also damage host cells with embedded or secreted antigen proteins.

Therefore, specific targeting of DCs represents the development direction for the next generation of mRNA vaccines, as it can simplify the production process, reduce production costs, improve vaccine safety, and enhance the immune efficacy of vaccines. Dendritic cell-specific intercellular adhesion molecule-3-grabbing nonintegrin (DC-SIGN) is a pattern recognition receptor and adhesion receptor on DCs, playing an important role in DC migration and adhesion, inflammatory responses, T cell activation, and initiation of immune responses. The wild-type Sindbis virus glycoprotein has two cell-binding sites, which can respectively target the DC-SIGN protein (a C-type lectin-like receptor capable of rapidly binding and phagocytosing substances, and an ideal target receptor on DCs) and cell surface heparan sulfate (expressed by various cell types). In existing technologies, by deleting amino acids at positions 61-64 in the E3 region of the SV-G protein, mutating positions 157KE158 in the E2 region to 157AA158, and inserting a 10-amino-acid tag sequence (MYPYDVPDYA) between amino acids at positions 71-74 in the E2 region, the protein is made unable to bind to heparan sulfate without affecting its ability to bind to DCs. Recombinant lentiviral vectors (LV) using Sindbis virus glycoprotein (SV-G) as a DC-SIGN ligand have the potential for better immune efficacy compared to non-specific LVs. However, risk factors such as integrative insertion mutations limit the clinical translation of LV vaccines. Additionally, the wild-type Sindbis virus glycoprotein, after engineering modifications to remove heparan sulfate-binding function, inherently suffers from poor protein stability and low binding efficiency to receptors, resulting in reduced binding specificity, making it difficult to apply in vaccine preparation. Moreover, unstable proteins are prone to accelerated degradation, exposing hidden antigenic epitopes and thereby triggering more immune responses. Although lipid nanoparticles (LNPs) can achieve DC-targeting specificity through conjugation with specific antibodies or ligands, there is currently a lack of evidence supporting the effectiveness of LNP-based DC-targeted mRNA vaccines.

### CONTENT OF THE INVENTION

To solve the above technical problems, the objective of the present invention is to provide a dendritic cell-targeted virus-like particle DVLP, and preparation method and application thereof. The virus-like particle (DVLP) is derived from the engineering modification of lentiviral vectors (LV) and can carry mRNA to specifically target DCs. In order to enhance the stability of the engineered SV-G protein and reduce spatial conflicts between atoms, the present invention mutates the amino acid tag MYPYDVPDYA inserted in the E2 region to AAAAYPYDVPDYA, which increases the protein stability without altering the protein structure or compromising DC targeting. Additionally, codon optimization is performed on the amino acids of the SV-G protein to adapt to expression in human cells.

The objective of the present invention can be achieved through the following technical solutions:
In the first aspect, the present invention provides a dendritic cell-targeted virus-like particle DVLP. The virus-like particle DVLP includes a virus-like particle (VLP) and Sindbis virus glycoprotein. The Sindbis virus glycoprotein is wrapped on a surface of the VLP, and an interior of the VLP encapsulates mRNA.

As an embodiment of the present invention, the amino acid sequence of the Sindbis virus glycoprotein (SV-G) is shown in SEQ ID NO.1. The SEQ ID NO.1 is obtained by optimizing the wild-type Sindbis virus glycoprotein (NP_062890.1) through engineering modifications: deleting amino acids at positions 61-64 in the E3 region of the SV-G protein, mutating positions 159KE160 in the E2 region to 159AA160, and inserting a 13-amino-acid tag sequence (AAAAYPYDVPDYA) between amino acids at positions 68-73 in the E2 region.

As an embodiment of the present invention, the mRNA includes at least one of spike mRNA, viral antigen mRNA, tumor antigen mRNA, and bacterial antigen mRNA.

Further, the mRNA is transcribed from genes of spike, viral antigens, tumor antigens, or bacterial antigens.

In some embodiments, the mRNA includes the spike mRNA, and gB1 mRNA and gD1 mRNA of HSV-1 virus.

In the second aspect, the present invention provides a preparation method of the dendritic cell-targeted virus-like particle DVLP, including the following steps:
S1, optimizing an amino acid sequence of an envelope protein of a wild-type Sindbis virus (reverse translating into a gene sequence and performing codon optimization suitable for expression in human cells) to obtain an SV-G gene;
S2, constructing an expression vector of the SV-G gene to obtain a pCMV-SV-G plasmid; and
S3, co-transfecting HEK293T cells with pCMV-SV-G, pRSV-REV, an antigen expression vector, pMDlg/PRRE-D64V, and pMS2S-PH-gagpol-D64V plasmids, collecting and purifying a cell culture supernatant after transfection.

As an embodiment of the present invention, in step S1, the nucleotide sequence of the SV-G gene is shown in SEQ ID NO.2. The SV-G gene sequence is a nucleotide sequence encoding the Sindbis virus glycoprotein.

In the present invention, the envelope protein of the wild-type Sindbis virus (i.e., the wild-type Sindbis virus glycoprotein) has a tendency to target DCs. After modification, the ability to target DCs is improved without affecting the virus titer.

As an embodiment of the present invention, constructing the expression vector of the SV-G gene in step S2 includes the following steps:
S2.1, integrating homology arms upstream and downstream of the SV-G sequence to obtain an artificially synthesized sequence;
S2.2, performing double enzyme digestion on the artificially synthesized sequence and a pMD2.G plasmid, and purifying and recovering; and
S2.3, ligating a recovered product using T4 DNA ligase to form the pCMV-SV-G plasmid.

As an embodiment of the present invention, in step S2.1, the artificially synthesized sequence is shown in SEQ ID NO.3.

As an embodiment of the present invention, in step S2, the nucleotide sequence of the pCMV-SV-G plasmid is shown in SEQ ID NO.4.

As an embodiment of the present invention, the antigen expression vector is pCMV-mRNA-mut-6×MS2. Where, the mRNA is selected from at least one of spike mRNA, viral antigen mRNA, tumor antigen mRNA, and bacterial antigen mRNA.

In some embodiments, the antigen expression vector includes pCMV-Spike-mut-6×MS2 and pCMV-gB1-gD1-6×MS2.

In the present invention, the construction methods of pRSV-REV, pCMV-Spike-mut-6×MS2, pMDlg/PRRE-D64V, and pMS2S-PH-gagpol-D64V refer to Patent CN112168958A; where, the nucleotide sequence of pRSV-REV is shown in SEQ ID NO.5; pCMV-Spike-mut-6×MS2 refers to pCMV-nCoV-S-6×MS2-mut-flag in this patent, and the nucleotide sequence of pCMV-Spike-mut-6×MS2 is shown in SEQ ID NO.6; pMDlg/PRRE-D64V refers to the integrase-deficient type of pMDlg/PRRE in this patent, and the nucleotide sequence of pMDlg/PRRE-D64V is shown in SEQ ID NO.7; pMS2S-PH-gagpol-D64V refers to the plasmid sequence expressing the lentiviral GagPol long-chain protein containing RNA-binding protein in this patent, and the nucleotide sequence of pMS2S-PH-gagpol-D64V is shown in SEQ ID NO.8.

Further, the construction of the antigen expression vector is carried out by replacing Spike in pCMV-Spike-mut-6×MS2 using an enzyme digestion and ligation method with other types of mRNA. The enzyme digestion and ligation method is a conventional technique in the art.

In the third aspect, the present invention provides an application of the dendritic cell-targeted virus-like particle DVLP in preparation of an mRNA vaccine.

Compared with the prior art, the present invention has the following beneficial effects:
1) The present invention optimizes the wild-type Sindbis virus glycoprotein (SV-G), removes the heparan sulfate binding ability, and retains the DC binding ability; in addition, the present invention optimizes the side chain conformation of SV-G, reduces the spatial conflict between atoms, thereby improving protein stability, further enhancing the binding effect with DC surface receptors, and thus improving DC targeting.
2) The present invention provides an mRNA vaccine capable of specifically targeting DCs *in vivo.*
3) A series of experiments in the present invention have proved that the virus-like particle can produce obvious co-localization with DCs in lymph nodes, demonstrating that the vaccine has extremely high DC targeting *in vivo.*
4) A series of experiments in the present invention have proved that after the injection of the virus-like particle, the mRNA carried by the virus-like particle is mainly concentrated at the injection site and lymph nodes, and the risk of spreading to non-target organs such as the spleen, liver, and kidneys is relatively small.
5) A series of experiments in the present invention have proved that after immunizing mice with the virus-like particle, it can induce high-titer neutralizing antibodies to resist viral infection and protect the host; at the same time, it also provides a platform for the development of tumor vaccines.

### DESCRIPTION OF THE DRAWINGS

Other features, objectives, and advantages of the present invention will become more apparent upon reading the detailed description of the non-limiting embodiments with reference to the following drawings:
FIG. 1 shows the AlphaFold3 modeling diagrams of Mutant Structure 1 and Mutant Structure 2 of the SV-G protein in Example 1;
FIG. 2 shows the mutant structure comparison of Mutant Structure 1 and Mutant Structure 2 of the SV-G protein in Example 1;
FIG. 3 shows the analysis result of spike-specific IgG antibody production of the DC-targeted virus-like particle (DVLP) and the non-specifically targeted virus-like particle (VSV-G VLP) via enzyme-linked immunosorbent assay (ELISA) experiment in Example 3;
FIG. 4 shows the analysis result of spike-specific IgG antibody production of the DC-targeted virus-like particle (DVLP) and lipid nanoparticle (LNP) via ELISA experiment in Example 3;
FIG. 5 shows the immunofluorescence images of the distribution of the DC-targeted virus-like particle (DVLP), non-specifically targeted virus-like particle (VSV-G VLP), and lipid nanoparticle (LNP) in mouse lymph nodes;
FIG. 6 shows the statistical analysis diagram of the biological distribution of spike mRNA of the DC-targeted virus-like particle (DVLP), non-specifically targeted virus-like particle (VSV-G VLP) and lipid nanoparticle (LNP) in different organs of mice and at the injection site (footpad);
FIG. 7 shows the detection diagram of serum neutralizing antibody titer in mice immunized with the DC-targeted virus-like particle (DVLP) (carrying HSV-1 gB1 mRNA and gD1 mRNA);
FIG. 8 shows the detection diagram of viral load in the skin and dorsal root ganglia of mice immunized with the DC-targeted virus-like particle (DVLP) (carrying HSV-1 gB1 mRNA and gD1 mRNA) after HSV-1 infection.

### SPECIFIC IMPLEMENTATIONS

The present invention will be described in detail below with reference to the drawings and specific examples. The following examples are implemented on the premise of the technical solution of the present invention, and provide detailed embodiments and specific operation processes, which will help those skilled in the art to further understand the present invention. It should be noted that the protection scope of the present invention is not limited to the following examples; several adjustments and improvements made under the premise of the concept of the present invention all fall within the protection scope of the present invention.

### Example 1

### Optimization Process Operation Steps and Mutation Stability Calculation

Engineering modification was carried out on the basis of the wild-type Sindbis virus glycoprotein SV-G (with the amino acid sequence shown in SEQ ID NO.9). Referring to the methods in the prior art, for the SV-G protein of the wild-type protein sequence NP_062890.1, the amino acids at positions 61-64 in the E3 region were deleted, the positions 159KE160 in the E2 region were mutated to 159AA160, and a 10-amino acid tag sequence (MYPYDVPDYA) was inserted between the amino acids at positions 68-73 in the E2 region; this was defined as Mutant Structure 1 (with the amino acid sequence shown in SEQ ID NO.18). The optimization scheme of the present invention is as follows: for the SV-G protein of the wild-type protein sequence NP_062890.1, the amino acids at positions 61-64 in the E3 region were deleted, the positions 159KE160 in the E2 region were mutated to 159AA160, and a 13-amino acid tag sequence (AAAAYPYDVPDYA) was inserted between the amino acids at positions 68-73 in the E2 region; this was defined as Mutant Structure 2. First, AlphaFold3 modeling analysis was performed: the amino acid sequences of SV-G of Mutant Structure 1 and Mutant Structure 2 were input respectively to model Mutant Structure 1 and Mutant Structure 2 individually, and then the Mutant Structures were compared. The structural conservation of the two was initially evaluated based on the overall conformation and Root Mean Square Deviation (RMSD). RMSD is an indicator used to quantify the difference between two structures (usually proteins or other macromolecules). A smaller RMSD value indicates greater similarity between the two structures, while a larger RMSD value indicates a greater difference between them. It measures the similarity or difference between the two by calculating the distance difference between the corresponding atomic positions in the structures. RMSD can be used to analyze whether a mutation will destroy the spatial structure of the protein. Then, Rosetta (using the default parameters of score_jd2) was further used to evaluate the stability of the two from the perspective of energy. The Ref2015 energy function was used to score the structures, and then the energy contribution items of the amino acid results were divided and analyzed in detail.

### Results:

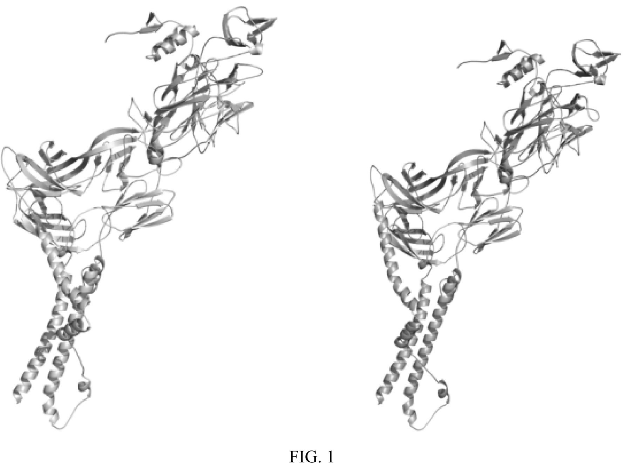

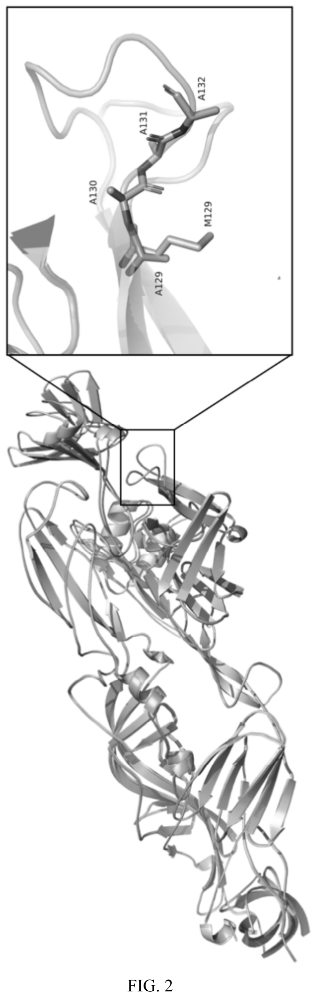

Modeling analysis of Mutant Structure 1 (hereinafter referred to as SV-G-M) and Mutant Structure 2 (hereinafter referred to as SV-G-4A; the 129th amino acid of this protein was mutated from M to AAAA, i.e., M129AAAA) of the Sindbis virus glycoprotein showed that the introduction of the M129AAAA mutation did not significantly affect the overall conformation of the protein (see FIG. 1, where the left panel shows SV-G protein Mutant Structure 1 and the right panel shows Mutant Structure 2). The RMSD of the two structures was 0.391 Å (an RMSD < 1 Å means the two structures are very similar and are usually considered almost identical), indicating that the predicted structure of SV-G-4A has high reliability, i.e., the mutation does not destroy the spatial structure of the protein. Comparison of the Mutant Structures showed that the mutation site was located in the loop region between the β-sheets at the top of the G protein, and the loop became longer after the mutation (FIG. 2). Rosetta was further used to evaluate the stability of the two from the perspective of energy. The Ref2015 energy function was used to score the structures. The results showed that the energy of SV-G-4A was significantly lower than that of SV-G-M, indicating that SV-G-4A had better stability (Table 1). After detailed division of the energy contribution items, it was found that SV-G-4A had better performance in terms of amino acid side chain conformation and the repulsive force between amino acid atoms. This indicated that the M129AAAA mutation optimized the side chain conformation of SV-G, reduced the spatial conflict between atoms, and thus improved the protein stability.

**Table 1 Rosetta Energy Calculation Results**

| Rosetta Score Term | SV-G-M | SV-G-4A |
|---|---|---|
| total_score | -689.402 | -772.894 |
| score | -689.401 | -772.894 |
| dslf_fa13 | 4.975 | 0.801 |
| fa_atr | -4225.031 | -4220.548 |
| fa_dun | 1100.689 | 1066.027 |
| fa_elec | -1046.336 | -1038.937 |
| fa_intra_rep | 6.015 | 6.022 |
| fa_intra_sol_xover4 | 123.986 | 123.33 |
| fa_rep | 876.433 | 829.212 |
| fa_sol | 2482.678 | 2473.057 |
| hbond_bb_sc | -78.22 | -75.931 |
| hbond_lr_bb | -287.253 | -288.051 |
| hbond_sc | -41.202 | -40.412 |
| hbond_sr_bb | -81.462 | -84.139 |
| linear_chainbreak | 0 | 0 |
| lk_ball_wtd | -65.808 | -70.039 |
| omega | 224.354 | 224.979 |
| overlap_chainbreak | 0 | 0 |
| p_aa_pp | -136.782 | -135.112 |
| pro_close | 88.582 | 91.517 |
| rama_prepro | 42.011 | 38.719 |
| ref | 322.969 | 326.61 |

| | | |
|---|---|---|
| Note: total_score: Total protein energy, which is the sum of all the following items. A lower value indicates better protein stability. fa_dun: Penalty score for amino acid side chain conformation. A higher value indicates that the protein contains more amino acids with unfavorable conformations. fa_rep: Penalty score for atomic repulsion between amino acids. A higher value indicates more atomic conflicts in the protein. | | |

Therefore, compared with Mutant Structure 1 of the Sindbis virus glycoprotein, the optimized Mutant Structure 2 has significantly reduced protein energy and improved stability. The main reasons are the more optimized side chain conformation and fewer spatial conflicts.

### Example 2

### Preparation of DC-Targeted Virus-Like Particle (DVLP)

The DC-targeted virus-like particle (DVLP) was obtained by modifying the envelope protein of a non-specifically targeted virus-like particle. The specific preparation steps are as follows:
S1, Based on Mutant Structure 2 in Example 1, the amino acid sequence of the envelope protein of the wild-type Sindbis virus was optimized to obtain the amino acid sequence of the optimized Sindbis virus glycoprotein. The amino acid sequence was reverse-translated into a DNA sequence, resulting in the nucleotide sequence encoding the amino acids of the Sindbis virus glycoprotein, which was named the SV-G gene sequence. The amino acid sequence of the envelope protein of the wild-type Sindbis virus is shown in SEQ ID NO.9.
S2, Homology arms were integrated upstream and downstream of the SV-G gene sequence to obtain an artificially synthesized sequence. AccI (1 µL) and KpnI (1 µL) were used to perform double enzyme digestion on the artificially synthesized sequence (2 µg) and the pMD2.G plasmid (2 µg) under the condition of 37°C for 2 h. The digested products were subjected to agarose gel electrophoresis, followed by purification and recovery. T4 DNA ligase was used to ligate the above recovered products to form a new plasmid (the SV-G gene sequence replaced the VSV-G gene of the known plasmid pMD.2G), which was designated as pCMV-SV-G. The nucleotide sequence of the pMD2.G is shown in SEQ ID NO.10. Among them, the integration of homology arms upstream and downstream of the SV-G sequence based on the DNA sequence of the pMD2.G plasmid is a conventional technique in the art.
S3, Plasmids including pCMV-SV-G (9.07 µg), pRSV-REV (7.26 µg), pCMV-Spike-mut-6×MS2 (31.46 µg), pMDlg/PRRE-D64V (15.73 µg), and pMS2S-PH-gagpol-D64V (15.73 µg) were sequentially added to a sterile 15 mL centrifuge tube. Ultrapure water was added to make up the volume to 1089 µL, followed by the addition of 1210 µL of 2×HEBS; the mixture was gently pipetted to mix well. Then 121 µL of CaCl₂ was added, and the mixture was gently pipetted again to mix well. After standing for 3 min and 20 s, the mixture was gently added dropwise to the cell culture supernatant for co-transfection of HEK293T cells. The cell culture supernatant was collected at 36 h and 60 h after transfection for subsequent purification.
S4, The collected supernatant was subjected to preliminary centrifugation under the conditions of 4°C and 3000×g for 10 min.
S5, The supernatant was filtered through a filter with a pore size of 0.45 µm. The filtered supernatant was added to an ultracentrifuge tube, with a volume of approximately 32 mL. Then a phosphate buffered saline (PBS) solution containing 20% (mass concentration) sucrose was slowly added to the bottom of the ultracentrifuge tube, with a volume of approximately 4 mL.
S6, The viral solution in S5 was subjected to ultracentrifugation for virus purification under the conditions of 4°C and 25,000 rpm for 2 h.
S7, After discarding the supernatant, 50 µL of PBS was added to soak and dissolve the virus at the bottom of the tube. After 2 h, the virus was collected to obtain the DC-targeted virus-like particle (DVLP), which was stored in a -80°C refrigerator.

### Example 3

### DC Targeting Test of VLP with Optimized Sindbis Virus Glycoprotein Pseudotyped Envelope, VSV-G Pseudotyped Envelope VLP, and Liposome Vector LNP

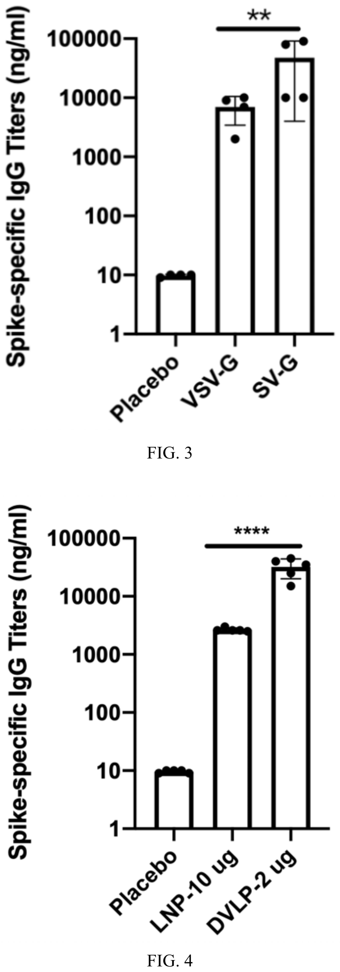

Mice (C57BL/6J mice) were immunized once via footpad injection with VSV-G VLP and DVLP (SV-G VLP, 1.5 µg), both carrying spike-mRNA. Mice in the negative control group (Placebo) were injected with an equal volume of PBS. Fourteen days after immunization, the mice were euthanized, and their serum was collected. The content of spike-specific IgG in the serum was detected using a mouse IgG ELISA detection kit (Bethyl, Montgomery, Texas; E99-131). In the ELISA experiment, the coating protein was replaced with recombinant spike protein (Novoprotein, DRA49), and the standard was replaced with anti-spike antibody (GeneTex, GTX632604). As shown in FIG. 3, under the condition of the same immunization dose, the envelope protein of DVLP (SV-G VLP) with specific DC targeting ability induced the body to produce more spike-specific IgG compared with the VLP modified with non-specifically targeted VSV-G envelope protein. This indicates that DVLP (SV-G VLP) has higher immune efficiency, which means the VLP with SV-G pseudotyped envelope has higher DC targeting ability.

Mice (C57BL/6J mice) were immunized once via footpad injection with LNP (10 µg) and DVLP (SV-G VLP, 2 µg p24), both carrying spike-mRNA (the VLP modified with DC-targeting envelope protein SV-G is referred to as DVLP). Mice in the negative control group (Placebo) were injected with an equal volume of PBS. Fourteen days after immunization, the mice were euthanized, and their serum was collected to detect the content of spike-specific IgG in the serum.

The results in FIG. 4 show that the level of spike-specific IgG induced by DVLP is significantly higher than that induced by traditional lipid nanoparticles (LNP). Therefore, DVLP has higher immune efficiency, which benefits from its DC targeting ability and mRNA delivery capacity.

### Example 4

### In Vivo Targeting Experiment of DC-Targeted Virus-Like Particle (DVLP)

### (1) Experimental Materials

6-week-old male C57BL/6J mice, DC-targeted virus-like particle (DVLP), non-specifically targeted virus-like particle (VSV-G VLP), lipid nanoparticle (LNP), anti-CD11c antibody (Servicebio, B11059), anti-spike antibody (Genetex, GTX632604), 488-conjugated donkey anti-rabbit secondary antibody (Jackson, 11-547-003), 555-conjugated goat anti-mouse secondary antibody (Huabio, HA1118), DAPI stain (Beyotime, P0131).

### (2) Experimental Methods

A. DVLP (2 µg), VSV-G VLP (2 µg), and LNP (10 µg) were injected into mice via footpad injection;
B. Twelve hours after injection, the mice were euthanized, and the lymph nodes were removed for fixation and subsequent frozen section processing;
C. The sections were permeabilized with Triton X-100 at a volume concentration of 0.4% under the condition of room temperature for 20 min;
D. The sections were blocked with a PBS solution containing 1% (mass concentration) bovine serum albumin (BSA) and 2% (volume concentration) goat serum under the condition of room temperature for 20 min;
E. Anti-CD11c antibody and anti-spike antibody were diluted with blocking buffer at a dilution ratio of 1:400, and then the incubation of the primary antibodies was performed at 4°C overnight;
F. The sections were washed with PBS for 3 times, 5 min each time;
G. 488-conjugated donkey anti-rabbit secondary antibody and 555-conjugated goat anti-mouse secondary antibody were diluted with PBS containing 1% (mass concentration) BSA at a dilution ratio of 1:800, and then the incubation of the secondary antibodies was performed at room temperature for 1 h;
H. The sections were washed with PBS for 3 times, 5 min each time; and
J. The cell nuclei were stained with DAPI, and the sections were mounted.

### (3) Experimental Results

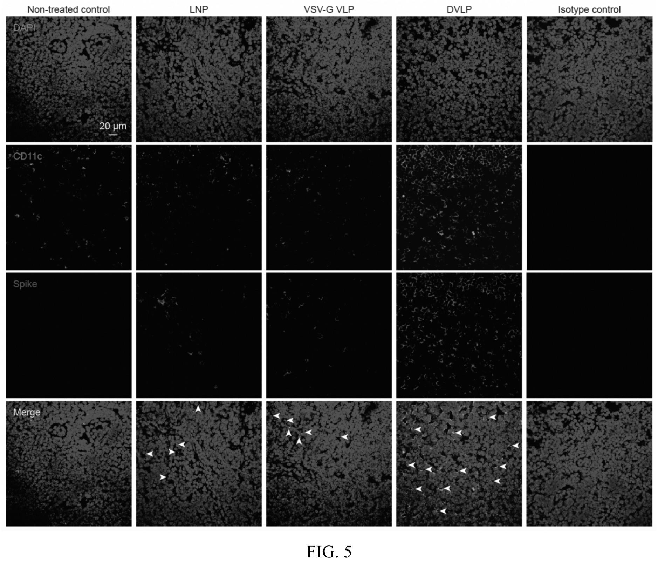

As shown in FIG. 5, the antigen (spike) expressed by DVLP highly colocalizes with the DC marker (CD11c) (indicated by arrows), which proves its DC targeting ability *in vivo.* The results show that compared with VSV-G VLP and LNP, DVLP can deliver more target mRNA to lymph nodes and achieve successful expression. At the same time, DVLP shows more colocalization with DC marker CD11c, which proves that DVLP has a good DC targeting function.

### Example 5

### In Vivo Distribution Experiment of DC-Targeted Virus-Like Particles (DVLP)

### (1) Experimental Materials

6-week-old male C57BL/6J mice, DC-targeted virus-like particle (DVLP), non-specifically targeted virus-like particle (VSV-G VLP), lipid nanoparticle (LNP), RNA isolater (Vazyme, R401-01), HiScript III RT SuperMix for qPCR (+gDNA wiper) (Vazyme, R323-01), and ChamQ Universal SYBR qPCR Master Mix (Vazyme, Q711).

### (2) Experimental Methods

A. DVLP (2 µg), VSV-G VLP (2 µg), and LNP (10 µg) were injected into mice via footpad injection;
B. Twelve hours after injection, the mice were euthanized, and the footpads, lymph nodes, livers, spleens, and kidneys were harvested;
C. RNA isolater was used to extract RNA from the footpads, lymph nodes, as well as the remaining livers, spleens, and kidneys;
D. HiScript III RT SuperMix for qPCR (+gDNA wiper) was used to reverse-transcribe the RNA obtained in step D into cDNA;
E. ChamQ Universal SYBR qPCR Master Mix was used for quantitative analysis of the cDNA. The sequences of the detection primers used included Spike-F (as shown in SEQ ID NO.11), Spike-R (as shown in SEQ ID NO.12), Mouse Gapdh-F (as shown in SEQ ID NO.13), and Mouse Gapdh-R (as shown in SEQ ID NO.14).

### (3) Experimental Results

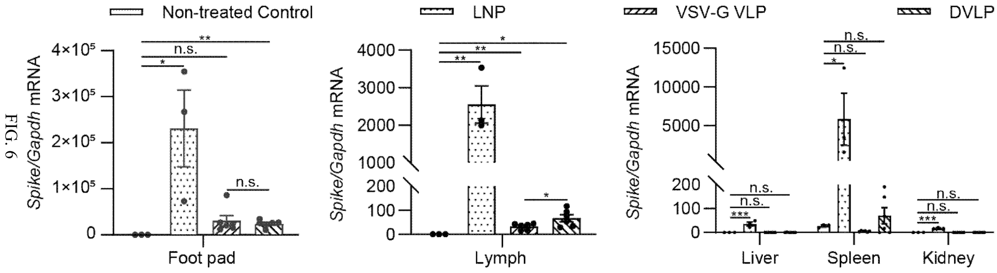

As shown in FIG. 6, compared with LNP, both DVLP and VSV-G VLP exhibited the characteristic of low off-target effects on organs, with the mRNA they delivered mainly concentrated at the injection site (footpad) and in the lymph nodes. Although there was no significant difference in the distribution of DVLP and VSV-G VLP in the footpads, the content of DVLP in the lymph nodes was significantly higher than that of VSV-G VLP. This indicates that DVLP has better lymph node targeting ability compared with VSV-G VLP.

### Example 6

### Effective Protection of Mice Against HSV-1 Infection via Delivery of HSV-1 gB1 mRNA and gD1 mRNA by DC-Targeted Virus-Like Particles (DVLP)

### (1) Experimental Materials

6-week-old male C57BL/6J mice, DC-targeted virus-like particle (DVLP), HSV-1 virus, Vero cells, low-melting-point agarose (Sangon, A600015), TaKaRa MiniBEST Viral RNA/DNA Extraction Kit (TaKaRa, 9766), and ChamQ Universal SYBR qPCR Master Mix (Vazyme, Q711).

### (2) Experimental Methods

A. On Day 0 and Day 14, DVLP (2 µg) carrying HSV-1 gB1 mRNA and gD1 mRNA was injected into mice via footpad injection for primary immunization and booster immunization, respectively. The construction method of DVLP carrying HSV-1 gB1 mRNA and gD1 mRNA refers to Example 1, with the only difference being that pCMV-Spike-mut-6×MS2 in Step S3 was replaced with pCMV-gB1-gD1-6×MS2; the sequence of pCMV-gB1-gD1-6×MS2 is shown in SEQ ID NO.15 in the sequence listing. Mice in the negative control group (Placebo/NC) were injected with an equal volume of PBS.
B. On Day 7 and Day 21, serum samples were collected from some mice for serum neutralization experiments. The serum was first diluted at a ratio of 1:10, followed by 2-fold serial dilutions until the final dilution ratio reached 1:1280. The diluted serum was thoroughly mixed with HSV-1 virus at a titer of 50 p.f.u, incubated at 37°C for 1 h, and then the mixture was added to Vero cells for 1 h of infection. After discarding the supernatant, 1% (mass concentration) low-melting-point agarose was added for further culture for 72 h, and the corresponding EC50 titer values were recorded.
C. On Day 28, the skin of mice near the spine on the back was depilated, and the skin surface was gently scratched 16 times with disposable sandpaper, followed by dropping 10 µL of HSV-1 at 10⁷ p.f.u for infection.
D. On Day 34, the infected skin and dorsal root ganglia (DRG) of mice were harvested separately. The virus was extracted using the TaKaRa MiniBEST Viral RNA/DNA Extraction Kit, and viral titer and viral genome were detected (quantitative analysis was performed using ChamQ Universal SYBR qPCR Master Mix). The sequences of the detection primers used included HSV-1 gD-F (as shown in SEQ ID NO.16), HSV-1 gD-R (as shown in SEQ ID NO.17), Mouse Gapdh-F (as shown in SEQ ID NO.13), and Mouse Gapdh-R (as shown in SEQ ID NO.14).

### (3) Experimental Results

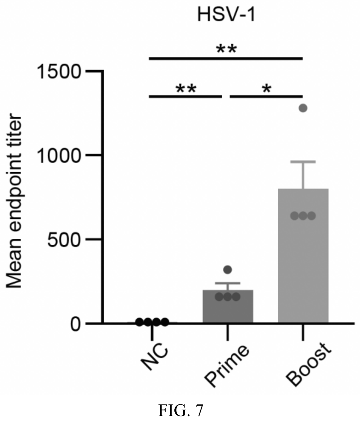

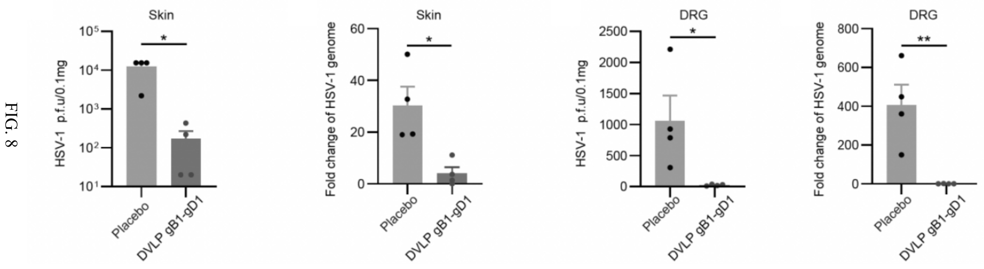

As shown in FIG. 7, primary immunization significantly induced the production of anti-HSV-1 neutralizing antibodies, and booster immunization further increased the titer of neutralizing antibodies. FIG. 8 shows that after two immunizations, the viral load in the skin tissue of mice was significantly reduced, and almost no virus was detected in the dorsal root ganglia, indicating that vaccination with DVLP gB1-gD1 mRNA has a strong neuroprotective effect.

The specific examples of the present invention have been described above. It should be understood that the present invention is not limited to the above specific embodiments, and those skilled in the art can make various modifications or changes within the scope of the claims, which do not affect the essence of the present invention.

## Claims

1. A dendritic cell-targeted virus-like particle DVLP, wherein the virus-like particle DVLP comprises a virus-like particle (VLP) and Sindbis virus glycoprotein, the Sindbis virus glycoprotein is wrapped on a surface of the VLP, and an interior of the VLP encapsulates mRNA.

2. The virus-like particle DVLP according to claim 1, wherein the amino acid sequence of the Sindbis virus glycoprotein is as shown in SEQ ID NO.1.

3. The virus-like particle DVLP according to claim 1, wherein the mRNA comprises at least one of spike mRNA, viral antigen mRNA, tumor antigen mRNA, and bacterial antigen mRNA.

4. A preparation method of the virus-like particle DVLP according to any one of claims 1-3, comprising the following steps:
S1, optimizing an amino acid sequence of an envelope protein of a wild-type Sindbis virus to obtain an SV-G gene;
S2, constructing an expression vector of the SV-G gene to obtain a pCMV-SV-G plasmid; and
S3, co-transfecting HEK293T cells with pCMV-SV-G, pRSV-REV, an antigen expression vector, pMDlg/PRRE-D64V, and pMS2S-PH-gagpol-D64V plasmids, collecting and purifying a cell culture supernatant after transfection.

5. The preparation method according to claim 4, wherein in the step S1, the nucleotide sequence of the SV-G gene is as shown in SEQ ID NO.2.

6. The preparation method according to claim 4, wherein constructing the expression vector of the SV-G gene in the step S2 comprises the following steps:
S2.1, integrating homology arms upstream and downstream of an SV-G sequence to obtain an artificially synthesized sequence;
S2.2, performing double enzyme digestion on the artificially synthesized sequence and a pMD2.G plasmid, and purifying and recovering; and
S2.3, ligating a recovered product using T4 DNA ligase to form the pCMV-SV-G plasmid.

7. The preparation method according to claim 6, wherein in the step S2.1, the artificially synthesized sequence is as shown in SEQ ID NO.3.

8. The preparation method according to claim 4, wherein in the step S2, the nucleotide sequence of the pCMV-SV-G is as shown in SEQ ID NO.4.

9. The preparation method according to claim 4, wherein the antigen expression vector is pCMV-mRNA-mut-6×MS2.

10. An application of the virus-like particle DVLP according to any one of claims 1-3 or a virus-like particle DVLP obtained by the preparation method according to any one of claims 4-9 in preparation of an mRNA vaccine.
